# EUROPÄISCHE PATENTSCHRIFT

(11) **EP 0 888 382 B1**
(45) Veröffentlichungstag und Bekanntmachung des Hinweises auf die Patenterteilung: **05.12.2001**
(21) Anmeldenummer: 97920517.6
(22) Anmeldetag: 14.03.1997
(51) Int. Cl.: C07K 1/16, C07K 7/64, B01D 15/02

(54) **CHROMATOGRAPHISCHES VERFAHREN ZUR GEWINNUNG VON HOCHGEREINIGTEM CYCLOSPORIN A UND VERWANDTEN CYCLOSPORINEN**
CHROMATOGRAPHIC PROCESS FOR OBTAINING VERY PURE CYCLOSPORIN A AND RELATED CYCLOSPORINS
PROCEDE CHROMATOGRAPHIQUE PERMETTANT D'OBTENIR DE LA CYCLOSPORINE A TRES PURE ET DES CYCLOSPORINES APPARENTEES

(30) Priorität: 21.03.1996 DE 19611094
(43) Veröffentlichungstag der Anmeldung: 07.01.1999
(73) Patentinhaber: Arzneimittelwerk Dresden GmbH, 01445 Radebeul (DE)
(72) Erfinder: VOIGT, Ullrich, D-01127 Dresden (DE); HEMPEL, Roland, D-01462 Mobschatz (DE); KINKEL, Joachim, D-55452 Guldental (DE); NICOUD, Roger-Marc, F-54502 Vandoeuvre-lès-Nancy Cédex (FR)
(86) Internationale Anmeldenummer: DE9700525
(87) Internationale Veröffentlichungsnummer: WO9734918

(56) Entgegenhaltungen:
- EP-A- 0 568 698
- US-A- 2 985 589
- US-A- 4 402 832
- US-A- 4 923 616
- AGRIC.BIOL.CHEM, Bd. 55, Nr. 4, 1991, Seiten 925-32, XP002036378 S.ADACHI ET AL.: "Separation of Peptide Groups with Definite Characteristics from Enzymatic Protein Hydrolysate"

## Beschreibung

Die vorliegende Erfindung betrifft ein neues Verfahren zur chromatographischen Aufreinigung von Cyclosporin A (Cy A) und verwandten Cyclosporinen, das für die Anwendung in der pharmazeutischen Industrie geeignet ist.
Dabei werden die Wirkstoffe zu ökonomisch günstigen Bedingungen in einer pharmazeutisch akzeptablen Reinheit erhalten, d.h. im Falle von Cyclosporin A werden beispielsweise die Reinheitsanforderungen der PHARMEUROPA (PHARMEUROPA Vol.4, No. 4, Seite 270, December 1992) erfüllt.

Mit der erfolgreichen Isolierung von Cyclosporin A aus Trichoderma polysporum (LINK ex PERS.) Rifai durch A. Rüegger und Mitarbeiter [Helv. Chim. Acta **59,** 112, (1976)] wurde erstmalig eine neue, stark immunsuppressive Substanzklasse zugänglich. Durch intensive Untersuchungen wurden in der Zwischenzeit mehr als 25 verwandte Cyclosporine mit immunsuppressiver und antifungischer Aktivität bekannt [R. Traber et al. Helv. Chim. Acta **70,** 13 (1987)].
Heutzutage ist die herausragende Bedeutung des Cyclosporin A als Mittel der Wahl bei der Unterdrückung der Immunabwehr nach Organtransplantationen unbestritten. Infolgedessen hat es in der Vergangenheit nicht an Anstrengungen gefehlt, um durch Verbesserung des Herstellungsverfahrens die ständig steigenden Anforderungen bezüglich Menge und Qualität für diesen lebensrettenden Arzneistoff zu erfüllen.
Die bisher bekannten technischen Lösungen zur Aufreinigung von Cyclosporin A-haltigen Rohextrakten beinhalten meist mehrere Chromatographiestufen unter Verwendung von organischen Lösungsmitteln als Elutionsmittel.
So wird in der oben erwähnten Arbeit von A. Rüegger zunächst an Kieselgel 60 Merck (0,063-0,2 mm) mit Chloroform bei steigenden Mengen Methanol chromatographiert. Anschließend wird das gewonnene Produkt an Sephadex LH 20 in Methanol einer Gelchromatographie unterworfen und danach schließlich an Aluminiumoxid (Brockmann, Akt. I) in Toluen mit steigenden Anteilen Ethylacetat chromatographiert.

Einer ähnlichen Verfahrensweise bedienen sich auch spätere Arbeiten (Tabelle 1). Häufig finden die Adsorbentien Sephadex LH 20, Kieselgel 60 Merck (0,063-0,2 mm) und Aluminiumoxid neutral Verwendung.
Als Fließmittel werden meist Gemische organischer Lösungsmittel eingesetzt.

Aufgrund ihrer hohen Toxizität sind dabei Chloroform und Methylenchlorid, sowohl bezüglich verbleibender Lösungsmittelreste im Wirkstoff als auch wegen der daraus resultierenden sicherheitstechnischen Probleme bei der Verarbeitung größerer Mengen dieser Stoffe (Destillation, Entsorgung), ungeeignet.

Ferner ist bei der Anwendung von Gradienten oder komplizierter, beispielsweise ternärer, isokratischer Mischungen eine neue Einstellung der Fließmittel nach der Destillation der Eluate umständlich und teuer.

Ähnlich ist auch eine von BIOGAL in dem kanadischen Patent CA 2 096 892 beschriebene Methode zu bewerten, bei welcher der Rohextrakt vor der Chromatographie einer Temperaturbehandlung unterworfen wird.
Das Rohprodukt wird hier ca.1 Stunde auf ca. 110°C erhitzt und anschließend definiert über 5 Stunden auf Raumtemperatur abgekühlt. Unter Einsatz eines hohen Anteils chlorierter Kohlenwasserstoffe werden in zwei nacheinander eingesetzten Fließmittelsystemen in einer einstufigen Chromatographie an Kieselgel 60 ca. 15% der aufgegebenen Menge Cyclosporin A in einer Reinheit von ca. 97,6% isoliert. Dieses Ergebnis kann jedoch im Hinblick auf eine industrielle Nutzung bezüglich Ausbeute und Reinheit des gewonnenen Wirkstoffes nicht befriedigen. Außerdem sollten diese komplizierten Naturstoffe wegen ihrer thermischer Instabilität und einer möglichen Isomerisierung erfahrungsgemäß keinem Temperaturstreß ausgesetzt werden.

Nach bisherigem Kenntnisstand kommen nur die Anmeldungen von FUJISAWA (WO 9 213094) und BIOGAL (CA 2 096 892) mit einer einstufigen chromatographischen Reinigung aus. Dazu ist allerdings eine aufwendige Fließmittelkombination bzw. ein Gradient erforderlich, was eine Fließmittelregenerierung erschwert.
Zur genaueren Beurteilung dieser Verfahren fehlen aber meistens Angaben über Ausbeuten und erzielte Produktreinheiten.

Beispiele für einige bisher bekannte chromatographische

Reinigungsmethoden für Cyclosporin A

| Patent | Firma | Reinigungsstufen |
|---|---|---|
| US 4 117 118 | SANDOZ | 1. Sephadex LH 20, Methanol |
| | | 2. Aluminiumoxid neutral, Toluen/Ethylacetat, Gradient |
| | | 3. Kieselgel 60, Chloroform/Methanol 98:2 |
| | | |
| US 4 215 199 | SANDOZ | 1. Kieselgel 60, Chloroform/Methanol 98:2 |
| | | 2. Sephadex LH 20, Methanol |
| | | 3. Kieselgel 60, Chloroform/Methanol 98:2 |
| | | |
| BE 879 402 | SANDOZ | 1. Sephadex LH 20, Methanol |
| | | 2. Kieselgel 60, Hexan/Aceton 66:33 |
| | | 3. Kristallisation, Aceton, -15°C |
| | | |
| WO 9 213 094 | FUJISAWA | 1. Kieselgel, Hexan Hexan/Ethylacetat, Gradient Aceton |
| | | |
| GB 2 227 489 | BIOGAL | 1. Kieselgel 60, Chloroform/Methanol/Aceton 92:4:4 |
| | | 2. Kieselgel 60, Hexan/Aceton, Gradient |
| | | |
| CA 2 096 892 | BIOGAL | 1. Kieselgel 60, Chloroform/Dichlormethan/ Ethanol 48:50:2 |
| | | |
| | | Chloroform/Ethylacetat/Ethanol 48:50:2 |

Eine kurze Beschreibung der SMB-Technik an sich findet sich z. B. bei R. M. Nicoud, LC-GC INTL Vol. 5, No. 5, 43-47. und K. K. Unger (Hrsg.), Handbuch der HPLC, Teil 2, GIT Verlag, Darmstadt, 1994. (siehe auch Abb. 1)

Ausgehend von dem hier geschilderten technischen Stand ergibt sich für eine erfinderische Lösung der chromatographischen Cyclosporin A-Reinigung mit hohen Ansprüchen an Produktreinheit und Ausbeute folgende Aufgabenstellung:
- Das neue Verfahren soll mehr als 70% der eingesetzten Cyclosporin A-Menge mit einer der PHARMEUROPA entsprechenden Qualität liefern (bezogen auf die chromatographischen Reinigunggsstufen).
- Das Verfahren soll höchsten Ansprüchen hinsichtlich Jahresdurchsatz genügen und gleichzeitig den Bedarf an Lösungsmitteln und Materialien für die stationären Phasen drastisch reduzieren
- Die technische Lösung soll einfach, schnell und robust sein, d.h. Lösungsmittel und Adsorbentien müssen über einen möglichst großen Zeitraum wiederverwendbar sein. Damit entfällt auch der Einsatz von schwierig einstellbaren bzw. regenerierbaren, isokratisch angewandten Lösungsmittelgemischen und Gradienten.
- Chlorierte Kohlenwasserstoffe sollen nicht verwendet werden.
- Das Verfahren soll Möglichkeiten zur Automatisierung, d.h. zur kontinuierlichen Betriebsweise bieten und gleichzeitig den Anforderungen einer GMP-gerechten Produktion genügen.

Als Ausgangsprodukt für die chromatographische Reinigung dient beispielsweise ein nach bekannten Methoden (z.B. DD 295 872 A5) aus Cyclosporin A-haltigen Trockenmycel durch Extraktion (Ethylacetat) und Entfettung (Petroleumbenzin / Methanol / Wasser) gewonnener Rohextrakt, der neben einer Reihe zumeist unbekannter gelb und rot gefärbter Substanzen sowie öliger Produkte beispielsweise folgende Zusammensetzung an Cyclosporinen aufweist:

| Cyclosporine | Unnorm. Relation %¹⁾ |
|---|---|
| C | 14,9 |
| B | 13,7 |
| L | 0,2 |
| A | 65,1 |
| G | 1,2 |
| D | 1,2 |
| Sonstiges | 3,7 |

| | |
|---|---|
| ¹⁾HPLC-analytische Bestimmung nach PHARMEUROPA Vol. 4, No. 4, 270 ff. | |

In einem ersten Chromtographieschritt werden die polaren Cyclosporine (C, B, L, U) von den unpolaren Cyclosporinen (G, D) geschieden, so daß zwei Wertfraktionen entstehen, die neben Cyclosporin A entweder nur polare oder nur unpolare Verunreinigungen enthalten. Dadurch kann in der zweiten Chromatographiestufe Cyclosporin A vorteilhaft von den jeweiligen Verunreinigungen gereinigt werden.

Erfindungemäß wird die Hochreinigung von Cyclosporin A mittels chromatographischer Reinigung durch Anwendung der konventionellen HPLC in Kombination mit der Simulated Moving Bed - Technik (SMB) wie folgt durchgeführt:
1. Chromatographie HPLC oder SMB-Technik
2. Chromatographie SMB-Technik

Siehe Schema 1 bis 4.

Genauer gesagt betrifft die Erfindung ein Verfahren zur Reinigung von Cyclosporin A und verwandten Cyclosporinen aus einem Cyclosporin-haltigen Rohextrakt unter Anwendung chromatographischer Verfahren mit Kieselgel als Adsorbens, welches sich dadurch auszeichnet, daß man Cyclosporin A und verwandte Cyclosporine aus einem Cyclosporin-haltigen Rohextrakt unter Anwendung chromatographischer Verfahren mit Kieselgel als Adsorbens
a) in einer ersten Chromatographiestufe mittels präparativer HPLC oder SMB-Technik den Rohextrakt durch Fraktionsschnitte im aufgetrennten Konzentrationsprofil in eine die unpolareren Begleitstoffe enthaltende Wertfraktion 1 und in eine die mehr polareren Begleitstoffe Wertfraktion 2 separiert und
b) die Wertfraktion 1 (Rafffinat) und die Wertfraktion 2 ( Extrakt) einer anschließenden zweiten Chromatographiestufe mittels SMB-Technik unterwirft. Dabei kann man sowohl die erste Chromatographiestufe als auch die zweite Chromatographiestufe im System Normalphase/Ethylacetat oder Umkehrphase, Acetonitril / Wasser durchführen.

Insbesondere durch den Einsatz der SMB-Technik werden folgende Vorteile erzielt:
• Es läßt sich eine absolut kontinuierliche Arbeitsweise realisieren, d.h.,durch den neuen Chromatographiemodus entfallen diskrete Substanzinjektionen. Eine solche kontinuierlich arbeitende Chromatographie ist vor allem für die industrielle Anwendung vorteilhaft.
• Die SMB Technik ermöglicht das Arbeiten mit konzentrierteren Lösungen als bisher. Dadurch sinkt der Lösungsmittelaufwand und damit gleichzeitig auch die benötigte Zeit für die Lösungsmittelrückgewinnung.
Aufgrund dieser günstigen ökonomischen Parameter wird die SMB-Technik in zunehmenden Maße auch für die chromatographische Trennung biotechnologisch produzierter Proteine ( Nadler, T.K., F.E. Sch.Sci., Purdue Univ. East Lafayette, IN 47907 USA) und Aminosäuren (Adachi, S. et.al.Agric. Biol. Chem. 1991,55, 925-32) eingesetzt.
Auch im vorliegenden Fall der Aufreinigung von Cyclosporin A -haltigen Produkten konnte die Überlegenheit der SMB im Vergleich mit konventionellen Chromatographieverfahren festggestellt werden.
• Vergleich SMB- mit HPLC-Technik

| **Phasensystem** | **Parameter** | **Quotient (SMB : HPLC)** |
|---|---|---|
| Normalphasensystem | Verbrauch Ethylacetat | 0,7 |
| | Verbrauch Si 60-Material | 0,25 |
| | Produktivität (g Aufgabe/Tag/kg Adsorbens) | 2,5 |
| | | |
| Umkehrphasensystem | Verbrauch Acetonitril/Wasser | 0,15 |
| | Verbrauch RP-18-Material | 0,15 |
| | Produktivität (g Aufgabe/Tag/kg Adsorbens) | 10 |

Wichtigste technische Voraussetzung für die Realisierung einer SMB-Trennung ist die genaue Einstellung der verschiedenen Teilflüsse (siehe Beispiele), um den quasi stationären Zustand der Elutionsfronten in Abhängigkeit von den Schaltzeiten zu gewährleisten.
Weiterhin ist für die optimale Einstellung der SMB-Trennung eine genaue Kenntnis der Adsorptionsisotherme vom Wertprodukt sowie von den Verunreinigungen im verwendeten Chromatographiesystem notwendig. Diese müssen vorher analytisch bestimmt werden

Weiterhin gelang es, durch Einführung einer sogenannten fünften Zone die bisher übliche Zwei-Komponenten-Trennung der klassischen Vier-Zonen-SMB zu verlassen. Durch das in dieser zusätzlichen Zone installierte Spülsystem wird jetzt im gleichen Lauf auch die Eliminierung von Verunreinigungen möglich, die bezüglich Cyclosporin A extreme k'-Werte aufweisen. Damit ist eine weitere Qualitätsverbesserung beim Wertprodukt möglich.
Üblicherweise können mit der Standard-SMB-Technik Gemische mit k'-Werten zwischen 0,6 und 2,0 meist gut getrennt werden (k' = 1 steht für das Wertprodukt). Jedoch werden Komponenten, die außerhalb dieses Bereiches liegen, normalerweise im Extrakt nur unvollständig ausgespült, so daß es zur Anreicherung im Raffinat gegenüber der Aufgabe (Feed) kommt (Abb. 1).
Nach der neuen erfindungsgemäßen Verfahrensweise werden die Säulen beim Umschalten zwischen der vierten und der ersten Zone durch Spülen mit einem Lösemittel starker Elutionskraft (z. B. Methanol) in einen definierten Zustand gebracht. Die betreffenden Säulen der Apparatur, die sich in dieser fünften Zone befinden, sind dann für die Dauer eines Taktes komplett aus dem geschlossenen Ringverband der übrigen vier Zonen herausgeschaltet (Abb. 1).

Diese fünfte Zone wird dabei in zwei Teilschritte unterteilt:
1. Während der Dauer des ersten Teilschrittes werden die Säulen der fünften Zone mit einem geeigneten Lösemittel hoher Elutionskraft gespült, damit die stationäre Phase von noch anhaftenden Verunreinigungen gereinigt wird.
   Als Spülmittel kommt bevorzugt Methanol zum Einsatz. Im Falle der Spülung des RP- Materials kann auch reines Acetonitril eingesetzt werden, wodurch sich das Problem der Lösemittelrückgewinnung durch den Wegfall eines zusätzlichen Lösemittels vereinfacht.
2. Im zweiten Teilschritt wird vom Spülmittel auf das für die jeweilige Trennung erforderliche Elutionsmittel umgespült.

Sind die Säulen so auf die Zonen verteilt, daß sich mehrere Säulen in der fünften Zone befinden, so kann der Spülvorgang intensiviert werden, wenn diese Säulen während des Spülens in Parallelschaltung gebracht werden.

Eine weitere Verfahrensoptimierung wurde bei der Hochreinigung der sogenannten Wertfaktion 2 der ersten Chromatographiestufe erreicht. Diese Fraktion enthält neben Cyclosporin A vor allem die polareren Verunreinigungen, wie Cyclosporin U und L. Hier konnte überraschenderweise nach Vertauschen der Entnahmestelle für Extrakt und Raffinat an der SMB-Anlage festgestellt werden, daß danach diese Wertfraktion 2 sehr gut getrennt werden kann. Hierbei werden die polaren Verunreinigungen vor dem Wertprodukt Cyclosporin A eluiert, d. h. sie weisen kürzere Retentionszeiten auf. Das bedeutet, daß bei Anwendung dieses speziellen SMB-Regimes in der zweiten Chromatographiestufe, in dieser Stufe dann ausschließlich mit dem RP-18-System gearbeitet werden kann, wodurch sich die Lösungsmittelrückgewinnung stark vereinfacht (Schema 1).
In den folgenden drei Beispielen, die die Eignung der SMB-Technik für die zweite Chromatographiestufe belegen, entsprechen die verwendeten Ausgangssubstanzen in ihrem Verunreinigungsprofil typischen Wertfraktionen, wie sie nach der ersten Chromatographiestufe, ausgeführt mit konventioneller präparativer HPLC. vorliegen.

In Beispiel 1 wird die Hochreinigung eines Zwischenproduktes der ersten Chromatographiestufe mittels SMB-Technik im Normalphasensystem Si 60 / Ethylacetat demonstriert. Das eingesetzte Produkt enthält neben Cyclosporin A vorwiegend polarere Verunreinigungen.
Im Ergebnis ist die deutliche Abreicherung der polaren Cyclosporine (insbesondere U und L sowie B und C) zu verzeichnen, wodurch die prinzipielle Eignung des Trennsystems in Verbindung mit der SMB-Technik verdeutlicht wird.

Das Beispiel 2 beschreibt die Hochreinigung eines Zwischenproduktes, welches als Wertfraktion 1 vorwiegend unpolare Verunreinigungen enthält, mittels SMB-Technik im System Umkehrphase RP-18/Acetonitril,Wasser (60:40, v/v). Nach der SMB Trennung ist eine deutliche Abreicherung der unpolaren Cyclosporine (insbesondere G und D) zu beobachten.

Im Beispiel 3 ist die Hochreinigung eines Zwischenproduktes, welches die Wertfraktion 1 mit vorwiegend unpolaren Verunreinigungen enthält, mittels SMB - Technik im System Normalphase / Ethylazetat beschrieben.
Im Vergleich zu Beispiel 1 erreicht man durch Vertauschen der Entnahmestellen für Extrakt / Raffinat einen höhere Abreicherung der unpolaren Verunreinigungen sowie eine Lösungsmitteleinsparung.
Diese Feintrennung ist insofern überraschend, da es bis vor Jahren noch, sogar mittels analytischer HPLC, nicht möglich war, diese chromatographisch außerordentlich ähnlichen Begleitsubstanzen von Cyclosporin A zu separieren.
Das erhaltene Cyclosporin A entspricht nach Umkristallisation sowohl den Qualitätsanforderungen der USP XXIII als auch der EUROPEAN PHARMACOPOEIA, 2. Edition 1995.

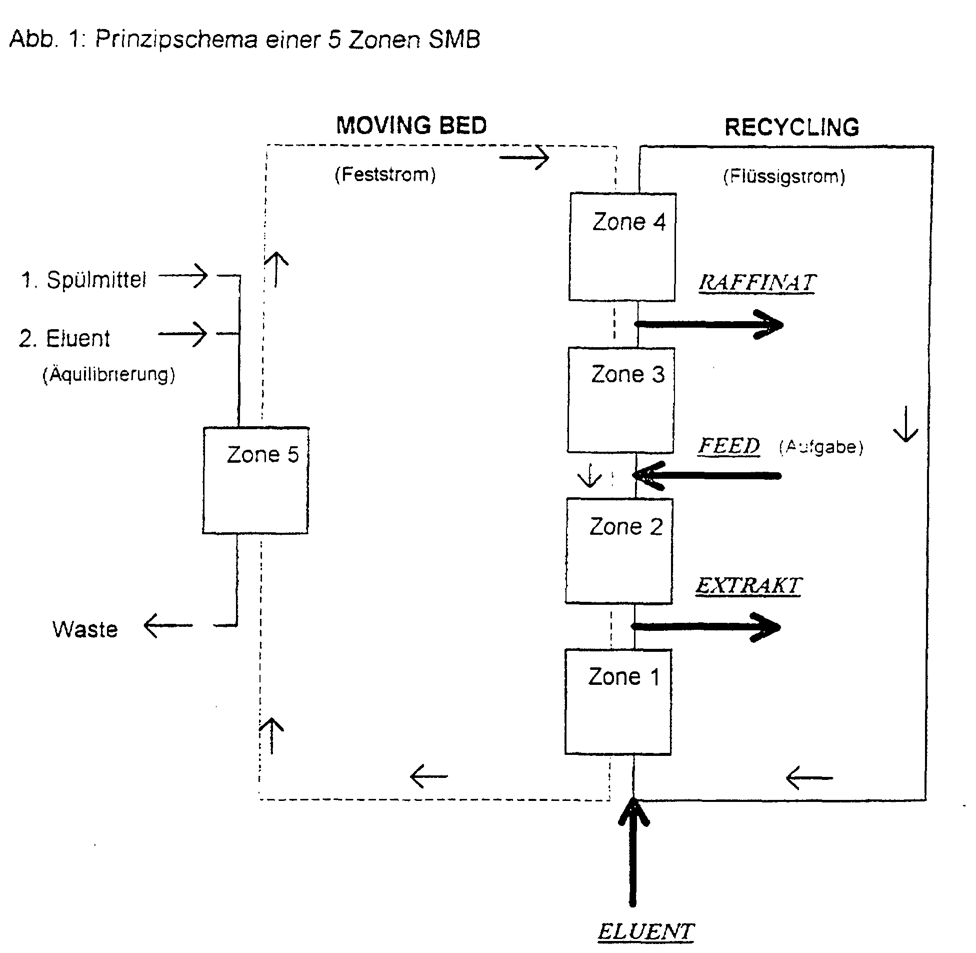

### Beschreibung von Abb. 1

Das Fließmittel wird zwischen Zone 1 und 4 im Kreis geführt. Die Probenaufgabe erfolgt zwischen den Zonen 2 und 3. Frischer Eluent wird zwischen den Zonen 4 und 1 aufgegeben. Raffinat (Cyclosporin A) wird zwischen Zone 3 und 4 und Extrakt (Cyclosporin A + Verunreinigungen) zwischen Zone 1 und 2 abgenommen. Jede Säule ist im Verbund mit vier Ventilen für Feed (Substanzaufgabe), Eluent, Extrakt (Wertprodukt + Verunreinigungen), und Raffinat (Wertprodukt) versehen. Die "Bewegung" des Trägermaterials wird durch Verschieben der Sammel- und Aufgabepunkte gegen die Elutionsrichtung simuliert. Dadurch läßt sich eine kontinuierliche Substanzverteilung zwischen den Phasen im Säulensystem erreichen und die Eluatkonzentrationen an den Abnahmepunkten erscheinen konstant.

Weiterhin konnte im experimentellen Test überraschend gefunden werden, daß die Abtrennung der Nebencyclosporine U und L einerseits sowie G und D andererseits durch die SMB-Technik vollständiger erfolgt als mit konventioneller Chromatographie und zugleich höhere Ausbeuten in den Stufen erreichbar sind. Als Folge daraus werden für vergleichbare Produktivitäten kleinere Anlagen möglich, die somit auch weniger Bedarf sowohl an Säulenfüllmaterial als auch an Elutionsmittel haben.

Natürlich ist prinzipell eine Anwendung der SMB-Technik auch in der ersten Chromatographiestufe möglich (Schema 3). Dabei kann prinzipiell beim Vorliegen geeigneter Rohextrakte (geringe Beladung mit Ballaststoffen, vor allem lipophiler Natur) auch in der ersten Stufe die SMB-Trennung im System Umkehrphase/Ace-tonitril, Wasser ausgeführt werden (Schema 4). Da, wie weiterhin gefunden wurde, auch polarere Verunreinigungen im System Umkehrphase/Acetonitril,Wasser nach Vertauschen der Raffinat-/Extrakt-Positionen von Cyclosporin A abgetrennt werden können, ist es dann auch möglich, die Cyclosporin A-Reinigung einzig unter Verwendung des Systems Umkehrphase/Acetonitril, Wasser zweistufig mittels SMB-Technik durchzuführen oder auch unpolarere Verunreinigungen im System Normalphase / Ethylacetat nach Vertauschen der Raffinat-/Extrakt-Positionen von Cyciosporin A abgetrennt werden können, ist es dann auch möglich, die Cyclosporin A-Reinigung einzig unter Verwendung des Systems Normalphase / Ethylacetat zweistufig mittels SMB-Technik durchzuführen.

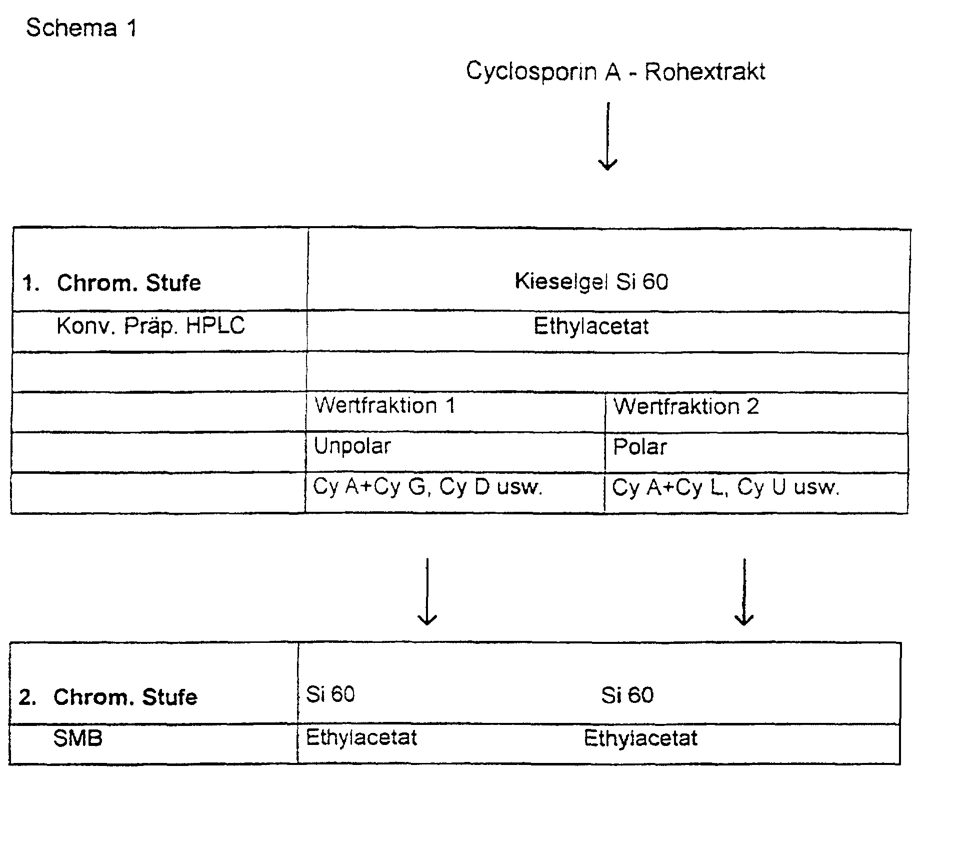

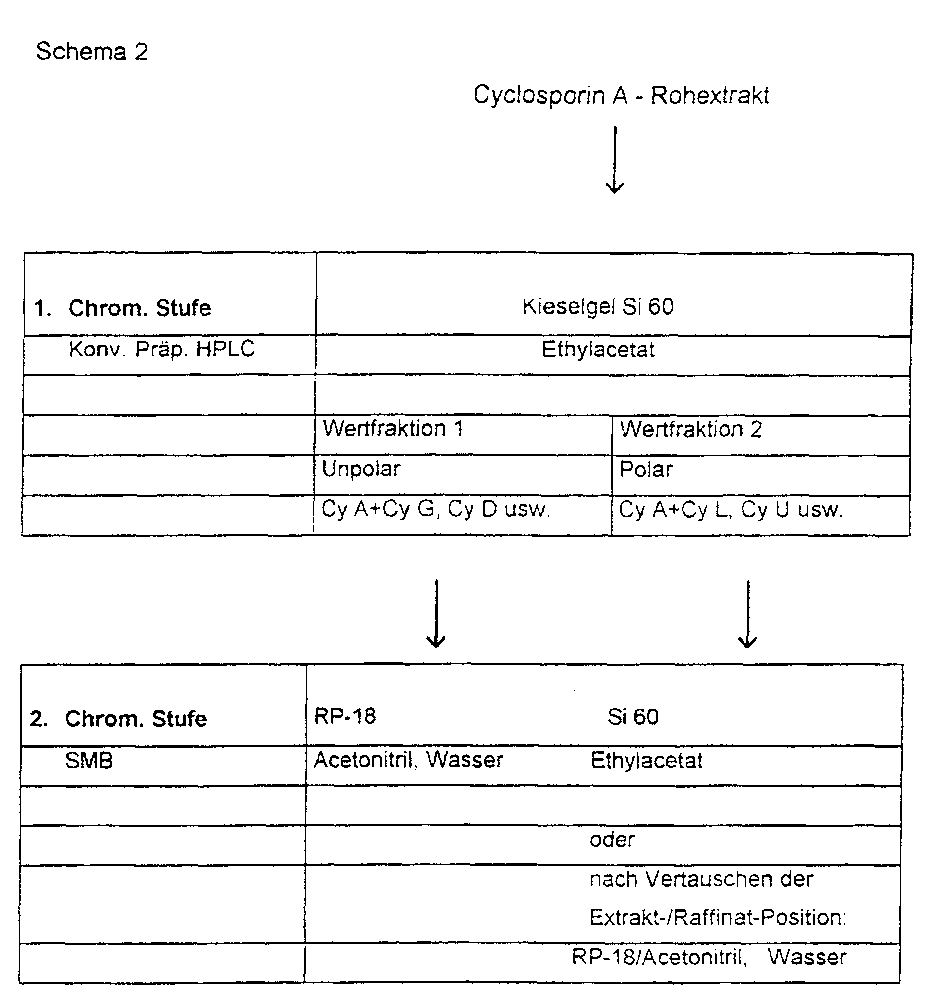

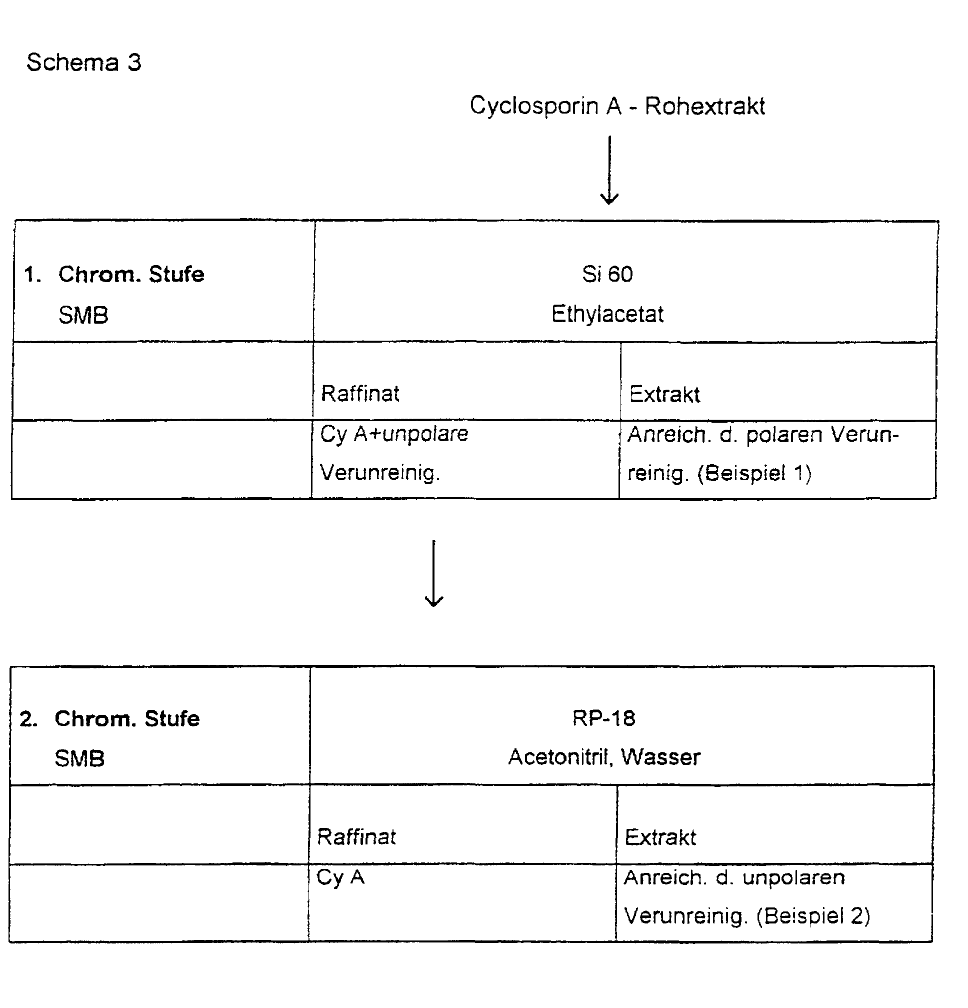

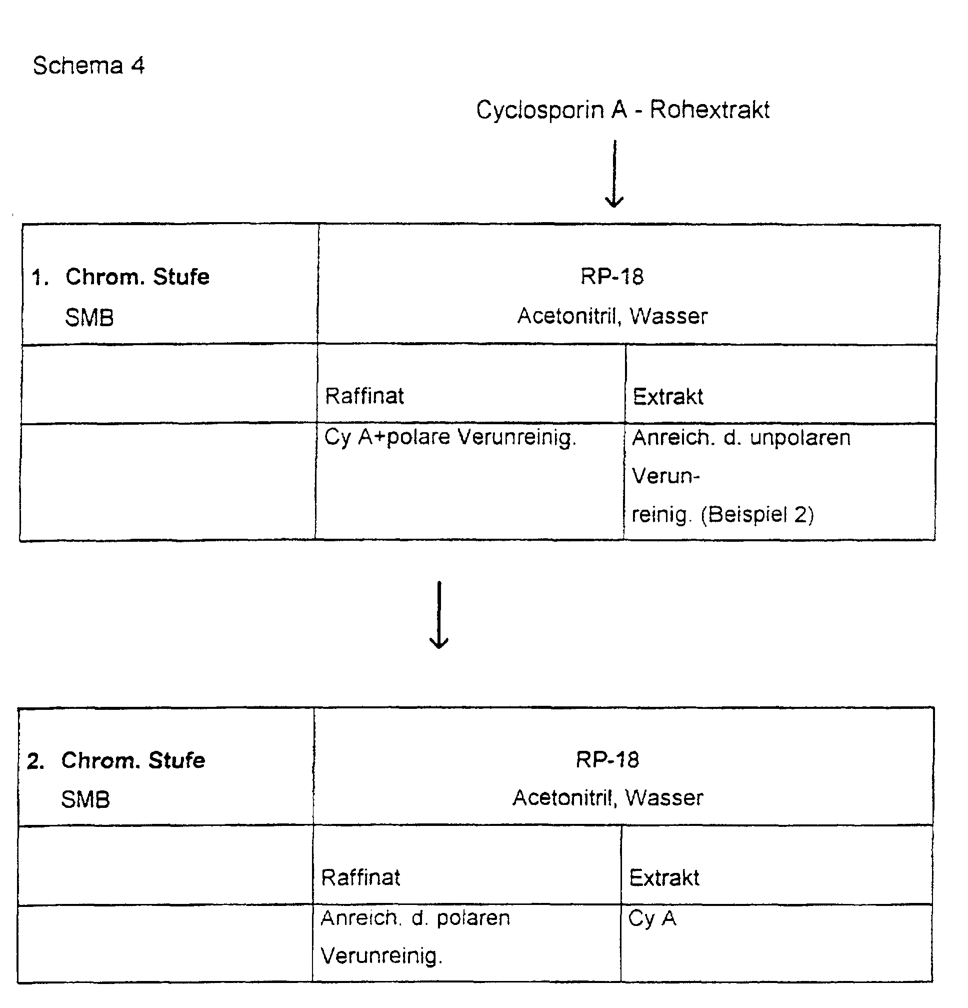

### Beispiel 1

Abtrennung überwiegend polarer Cyclosporine (Cyclosporine C, B, L, U) vom Cyclosporin A in der zweiten Chromatographie mit Hilfe der SMB-Technik im System Normalphase/Ethylacetat.

| Anlage | LiChrosep® 8 - 50, Pilotanlage | |
|---|---|---|
| Säule | 8 Säulen, Länge 100mm x 50mm InnenØ, axiale Kompression | |
| Stationäre Phase | LiChrospher® Si 60, 15 µm | |
| Mobile Phase | Ethylacetat | |

| Rohsubstanz | # 011194 Cyclosporine | Reinheit [%] |
|---|---|---|
| | C | 2,3 |
| | B | 6,9 |
| | L | 0,7 |
| | U | 1,2 |
| | A | 86,2 |
| | G | 1,0 |
| | D | 0,1 |
| | Summe der Verunreinigungen | 13,8% |
| Substanzaufgabe Feed | Lösung in Ethylacetat 5,8 g/l 5,3 ml/min | |
| Eluent | 100 ml/min | |
| Recycling | 151 ml/min | |
| Detektion | HPLC-Analyse im Extrakt- und Raffinatstrom | |
| Ergebnisse | | |

| Raffinat | Cyclosporine | Reinheit [%] |
|---|---|---|
| | C | 0,2 |
| | B | 0,4 |
| | L | 0,0 |
| | U | 0,4 |
| | A | 97,4 |
| | G | 1,1 |
| | D | 0,1 |
| | Summe der Verunreinigungen: | 2,6% |
| | Ausbeute, Cyclosporin A: | > 95 % |
| Extrakt | Cyclosporin A | 75,5 % |
| | Summe der Verunreinigungen: | 24,5 % |
| Das Ergebnis des Experiments zeigt die prinzipielle Möglichkeit der Abtrennung vor allem der polaren Verunreinigungen vom Cyclosporin A im System Si 60 / Ethylacetat. | | |

### Beispiel 2

Abtrennung überwiegend unpolarer Cyclosporine (Cyclosporine G, D) vom Cyclosporin A mit Hilfe der SMB-Technik im System RP-18/Acetonitril,Waser.

| Anlage | LiChrosep® 8 - 50, Pilotanlage | |
|---|---|---|
| Säule | 8 Säulen, Länge 100mm x 50mm InnenØ, axiale Kompression | |
| Stationäre Phase | LiChrospher® RP-18, 15 µm | |
| Mobile Phase | Acetonitril / Wasser - 60 / 40 - v / v | |

| Rohsubstanz | # 251094 Cyclosporine | Reinheit [%] |
|---|---|---|
| | L | 0,3 |
| | U | 0,8 |
| | A | 92,5 |
| | G | 4,1 |
| | D | 1,6 |
| | Summe der Verunreinigungen : | 7,5% |
| Substanzaufgabe | Lösung in Acetonitril | |
| Feed | 1 g/l | |
| | 12,7 ml/min | |
| Eluent | 81 ml/min | |
| Recycling | 151 ml/min | |
| | | |
| Detektion | HPLC-Analyse im Extrakt- und Raffinatstrom | |
| Ergebnisse | | |

| Raffinat | Cyclosporine | Reinheit [%] |
|---|---|---|
| | Unbekannt (α = 3,4) | 0,1 |
| | L | 0,2 |
| | U | 0,5 |
| | A | 99,1 |
| | G | 0,0 |
| | D | 0,0 |
| | Unbekannt (α = 20,1) | 0,1 |
| | Summe der Verunreinigungen : | 0,9% |
| | Gehalt, Cyclosporin A (getr. Substanz) : | 99,4 % |
| | Stufenausbeute Cyclosporin A : | > 95 % |
| Die im Experiment erzielte Reinheit des Raffinates beträgt 99,1 %. Nach der Trocknung dieser Substanz, wurde ein Cyclosporin A-Gehalt von 99,4% bestimmt. Das Ergebnis zeigt die Möglichkeit der Abtrennung der unpolaren Verunreinigungen vom Cyclosporin A im System RP-18/Acetonitril, Wasser (60:40,v/v). | | |

### Beispiel 3

Abtrennung überwiegend unpolarer Cyclosporine (Cyclosporine C, G, D) vom Cyclosporin A in der zweiten Chromatographie mit Hilfe der SMB-Technik im System Normalphase/Ethylacetat durch Vertauschen der Extrakt-und Raffinat - Entnahmestelle.

| Anlage | LiChrosep® 12 - 26, Pilotanlage | |
|---|---|---|
| Säule | 8 Säulen, Länge 100mm x 26mm InnenØ, axiale Kompression | |
| Stationäre Phase | LiChrospher® Si 60, 15 - 25 µm | |
| Mobile Phase | Ethylacetat | |

| Rohsubstanz | Cyclosporine | Reinheit [%] |
|---|---|---|
| | C | 0,04 |
| | B | 0,122 |
| | L | 0,075 |
| | A | 92,462 2,934 |
| | G | |
| | D | 4,177 |
| | Summe der Verunreinigungen | 7,538% |
| Substanzaufgabe | Lösung in Ethylacetat 28 g/l | |
| Feed | 1,5 ml/min | |
| Eluent | 16,4 ml/min | |
| Detektion | HPLC-Analyse im Extrakt- und Raffinatstrom | |
| Ergebnisse | | |

| Extrakt | Cyclosporine | Reinheit [%] |
|---|---|---|
| | C | 0,02 |
| | B | 0,075 |
| | L | 0,063 |
| | A | 99,364 |
| | unbek. | 0,219 |
| | G | 0,175 |
| | Summe der Verunreinigungen: | 0,636% |
| | Ausbeute, Cyclosporin A: | > 95 % |
| Das Ergebnis zeigt, daß durch das o.g. Schaltregime durch die SMB-Technik auch unpolare Verunreinigungen sehr gut im System Si 60 / Ethylacetat abgetrennt werden können. | | |

## Patentansprüche

1. Verfahren zur Reinigung von Cyclosporin A und verwandten Cyclosporinen aus einem Cyclosporin-haltigen Rohextrakt unter Anwendung chromatographischer Verfahren mit Kieselgel als Adsorbens **dadurch gekennzeichnet, daß** man
a) in einer ersten Chromatographiestufe mittels präparativer HPLC oder "simulated moving bed"-Technik den Rohextrakt durch Fraktionsschnitte im aufgetrennten Konzentrationsprofil in eine die unpolareren Begleitstoffe enthaltende Wertfraktion 1 und in eine die mehr polareren Begleitstoffe Wertfraktion 2 separiert und
b) die Wertfraktion 1 (Rafffinat) und die Wertfraktion 2 ( Extrakt) in einer anschließenden zweiten Chromatographiestufe einer weiteren Reinigung mittels SMB-Technik unterwirft.

2. Verfahren nach Anspruch 1, **dadurch gekennzeichnet, daß** man
a) sowohl die erste Chromatographiestufe als auch die zweite Chromatographiestufe im System Normalphase/Ethylacetat oder Umkehrphase, Acetonitril / Wasser durchführt.
b) die Wertfraktion 1 (Rafffinat) mittels SMB-Technik im System Umkehrphase. Acetonitril / Wasser und die Wertfraktion 2 ( Extrakt) einer zweiten Chromatographiestufe im System Normalphase/Ethylacetat unterwirft.
c) die Wertfraktion 2 (Extrakt) mittels SMB-Technik im System Umkehrphase, Acetonitril / Wasser und die Wertfraktion 1 (Raffinat) einer zweiten Chromatographiestufe im System Normalphase/Ethylacetat unterwirft.

3. Verfahren nach Anspruch 1, **dadurch gekennzeichnet, daß** man mittels Einführung einer fünften Zone einen Spülschritt zuerst mit einem Alkohol und anschließend mit dem Fließmittel durchführt, und daß bei mehreren Säulen in der fünften Zone diese in Parallelschaltung durchströmt werden.

4. Verfahren nach Anspruch 2 **dadurch gekennzeichnet, daß** man die Trennung in der zweiten Chromatographiestufe im System Umkehrphase Acetonitril/Wasser mit einem Verhältnis Acetonitril/Wasser von 40 bis -80 zu 20 bis 60, vorzugsweise 60:40 (v/v) durchführt.

5. Verfahren nach Anspruch 1 und 2, **dadurch gekennzeichnet, daß** man die Säulen und das Fließmittel in der SMB-Anlage im Temperaturbereich von 40 bis 80 °C vorzugsweise aber bei 60 °C hält.

6. Verfahren nach Anspruch 2 **dadurch gekennzeichnet, daß** man im System Umkehrphase/Acetonitril, Wasser den p_{H}-Wert des Fließmittels im Bereich von 2 bis 5, jedoch vorzugsweise auf 3 einstellt .

## Claims

1. Process for the purification of cyclosporin A and related cyclosporins from a cyclosporin-containing crude extract using chromatographic processes with silica gel as adsorbent, **characterized in that**
a) in a first chromatographic stage, the crude extract is separated by fraction cuts in the separated concentration profile into a useful fraction 1 containing the non-polar concomitants and into a useful fraction 2 [lacuna] the more polar concomitants by means of preparative HPLC or "simulated moving bed" technique and
b) the useful fraction 1 (raffinate) and the useful fraction 2 (extract) are subjected to a further purification in a subsequent second chromatographic stage by means of SMB technique.

2. Process according to Claim 1, **characterized in that**
a) both the first chromatographic stage and the second chromatographic stage are carried out in the system normal-phase/ethyl acetate or reverse-phase, acetonitrile/water [sic].
b) the useful fraction 1 (raffinate) is subjected to a second chromatographic stage in the reverse-phase system, acetonitrile/water and the useful fraction 2 (extract) is subjected to a second chromatographic stage in the normal-phase system/ethyl acetate [sic] by means of SMB technique.
c) the useful fraction 2 (extract) is subjected to a second chromatographic stage in the reverse-phase system, acetonitrile/water and the useful fraction 1 (raffinate) is subjected to a second chromatographic stage in the normal-phase system/ethyl acetate [sic] by means of SMB technique.

3. Process according to Claim 1, **characterized in that**, by means of introduction of a fifth zone, a rinsing step is carried out first with an alcohol and then with the eluent, and **in that** with a plurality of columns in the fifth zone these are flowed through in parallel.

4. Process according to Claim 2, **characterized in that** the separation in the second chromatographic stage is carried out in the reverse-phase system acetonitrile/water using an acetonitrile/water ratio of 40 to -80 [sic]:20 to 60, preferably 60:40 (v/v).

5. Process according to Claim 1 and 2, **characterized in that** the columns and the eluents in the SMB plant are kept in the temperature range from 40 to 80°C, but preferably at 60°C.

6. Process according to Claim 2, **characterized in that** the pH of the eluent in the reverse-phase system/ acetonitrile, water [sic] is set in the range from 2 to 5, but preferably at 3.

## Revendications

1. Procédé pour la purification de cyclosporine A et de cyclosporines apparentées, à partir d'un extrait brut contenant de la cyclosporine, au moyen d'un procédé chromatographique avec du gel de silice en tant qu'adsorbant,
**caractérisé en ce que**
a) dans une première étape de chromatographie, on fractionne par HPLC préparative ou la technique de "simulated moving bed" (SMB, lit mobile simulé) l'extrait brut par fractions dans le profil de concentrations séparées, en une fraction de valeur 1 contenant les impuretés non polaires et en une fraction de valeur 2 contenant les impuretés plus polaires, et
b) on soumet la fraction de valeur 1 (raffinat) et la fraction de valeur 2 (extrait) à une purification plus poussée, dans une seconde étape de chromatographie subséquente, au moyen de la technique SMB.

2. Procédé selon la revendication 1,
**caractérisé en ce que**
a) on effectue aussi bien la première étape de chromatographie que la seconde étape de chromatographie dans le système phase normale/acétate d'éthyle ou phase inversée acétonitrile/eau,
b) on soumet la fraction de valeur 1 (raffinat) au moyen de la technique SMB dans le système phase inversée acétonitrile/eau et la fraction de valeur 2 (extrait) à une seconde étape de chromatographie dans le système phase normale/acétate d'éthyle,
c) on soumet la fraction de valeur 2 (extrait) au moyen de la technique SMB dans le système phase inversée acétonitrile/eau et la fraction de valeur 1 (raffinat) à une seconde étape de chromatographie dans le système phase normale/acétate d'éthyle.

3. Procédé selon la revendication 1,
**caractérisé en ce que**
- par introduction d'une cinquième zone, on effectue une étape de lavage d'abord avec un alcool et ensuite avec l'éluant, et
- dans le cas de plusieurs colonnes dans la cinquième zone, celle-ci sont traversées en montage en parallèle.

4. Procédé selon la revendication 2,
**caractérisé en ce que**
dans la seconde étape de chromatographie on effectue la séparation dans le système phase inversée acétonitrile/eau avec un rapport acétonitrile/eau de 40:60 à 80:20, de préférence, de 60:40 (v/v).

5. Procédé selon les revendications 1 et 2,
**caractérisé en ce que**
dans l'unité de SMB on maintient les colonnes et l'éluant dans la plage de températures de 40 à 80°C, mais de préférence à 60°C.

6. Procédé selon la revendication 2,
**caractérisé en ce que**
dans le système phase inversée acétonitrile/eau on ajuste le pH de l'éluant dans l'intervalle de 2 à 5, mais de préférence à 3.
